# EUROPEAN PATENT APPLICATION

(11) **EP 3 162 367 A1**
(43) Date of publication of application: **03.05.2017**
(21) Application number: 15811980.0
(22) Date of filing: 16.02.2015
(51) Int. Cl.: A61K 31/375, A61K 31/6615, A61K 47/22

(54) **COMPOSITION FOR SKIN PENETRATION, CONTAINING CATIONIC MOLECULE TRANSPORTER AND ANIONIC BIOACTIVE MATERIAL**

(30) Priority: 27.06.2014 KR 20140079931
(71) Applicant: Postech Academy-Industry Foundation, Pohang-si, Gyeongsangbuk-do 790-784 (KR)
(72) Inventor: CHUNG, Sung-Kee, Gyeongju-si Gyeongsangbuk-do 780-240 (KR); LEE, Woo Sirl, Pohang-si Gyeongsangbuk-do 790-751 (KR)
(74) Representative: dompatent von Kreisler Selting Werner - Partnerschaft von Patent- und Rechtsanwälten mbB
(86) International application number: PCT/KR2015/001574
(87) International publication number: WO 2015/199309

(57) **Abstract**

The present invention relates to a composition for skin penetration, wherein the composition contains an ion conjugate in which a cationic compound is ionically combined with an anionic bioactive material, and delivers the bioactive material into the skin. According to the present invention, various kinds of anionic bioactive materials, such as diagnostic reagents, medicines, and fluorescent materials, are allowed to easily pass through cell membranes and skin layers, and thus can be delivered to the epidermal layer and dermal layer of the skin.

## Description

### FIELD OF THE INVENTION

The present invention relates to a composition comprising a cationic molecular transporter and an anionic physiologically active material for skin penetration, in particular, for delivering the physiologically active material into the epidermis layer and a part of the dermis layer.

### BACKGROUND OF THE INVENTION

A cell is a basic unit of all living organisms, and is composed of the cytoplasm in which intracellular organelles are present and the cell membrane which protects the cytoplasm as well as separates it from the environment. Since the cell membrane is a selectively permeable barrier composed of a phospholipid bilayer and proteins of fluid state distributed in the bilayer in mosaic, it limits the passage of many useful therapeutic agents. Especially, hydrophilic molecules, highly charged molecules of low molecular weights and macromolecules such as peptides and oligonucleotides, e.g., nucleic acid or gene, cannot be transported across the cell membrane. Although special methods for transporting the molecules into cells have been developed (*see* [S. Futaki, Adv. Drug Delivery Rev., 2005, 57, 547-558] and [P. A. Wender, et al., Adv. Drug Delivery Rev., 2008, 60, 452-472]), they have raised many problems including cytotoxicity.

In the meantime, a number of molecular transporters for delivering physiologically active molecules across biological membranes have been developed (*see* [S. K. Chung, et al., Int. J. Pharmaceutics, 2008, 354, 16-22]; [K. K. Maiti, et al., Angew. Chem. Int. Ed., 2007, 46, 5880-5884]; [K. K. Maiti, et al., Angew. Chem. Int. Ed., 2006, 45, 2907-2912]; and Korean Patent Nos. 10-0578732, 10-0699279, 10-0849033 and 10-1021078).

However, no attempt has been made to deliver water-soluble anionic physiologically active materials into the skin by using such molecular transporters. In order to deliver physiologically active materials having a phosphate group or a carboxylate group such as ascorbic acid phosphate or salicylic acid known as non-steroidal anti-inflammatory drugs into the skin, the present inventors have prepared an ionic complex through ionic bonding of physiologically active materials and known molecular transporters at a specific ratio, and found that the prepared ionic complex can enhance skin permeability, and thus accomplished the present invention.

### SUMMARY OF THE INVENTION

It is an object of the present invention to provide a composition for skin penetration, which is for delivering a physiologically active material into the skin.

In accordance with one aspect of the present invention, there is provided a composition for skin penetration, comprising an ionic complex in which a cationic compound of any one selected from the following compounds represented by formulae (1) to (4) and an anionic physiologically active material are combined through ionic bonding, the composition being used for delivering the physiologically active material into the skin: 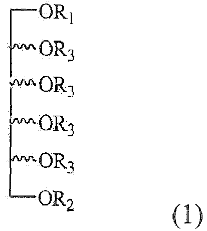 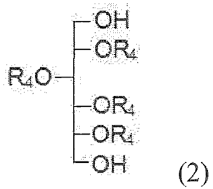 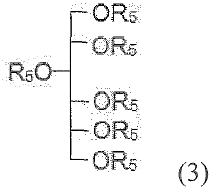 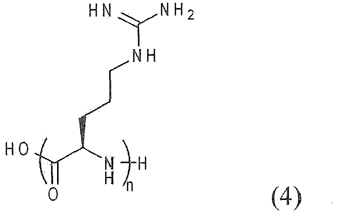 wherein
R₁ and R₂ are each independently H, C₁-C₆ alkyl, C₆-C₁₂ aryl C₁-C₆ alkyl, C₃-C₈ cycloalkyl, C₃-C₈ heteroalkyl, -(CH₂)ₘNHR', -(CH₂)₁CO₂R", -COR"', - SO₂R"", or a physiologically active material to be transported, where R', R", R"' and R"" are each independently H, C₁-C₆ alkyl, C₆-C₁₂ aryl C₁-C₆ alkyl, C₃-C₈ cycloalkyl, or C₃-C₈ heteroalkyl, m is an integer in the range of 2 to 5, and l is an integer in the range of 1 to 5;
R₃ is 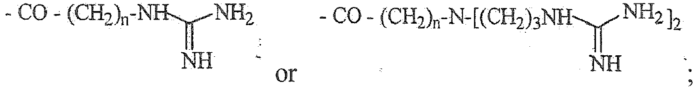
R₄ is 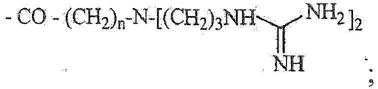
R₅ is 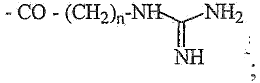 and
n is an integer in the range of 1 to 12.

The composition according to the present invention has an excellent effect of transporting molecules having difficulty in passing through cell membranes or skin layers, e.g., ascorbic acid phosphate, aspirin, water-soluble anionic physiologically active materials, into the skin, thus may be effectively used for delivering physiologically active materials into the skin.

### BRIEF DESCRIPTION OF THE DRAWINGS

The above and other objectives and features of the present invention will become apparent from the following description of the invention, when taken in conjunction with the accompanying drawings, in which:
Fig. 1A shows fluorescence images of cells treated with each of fluorescein (FITC)-labeled uridine diphosphate N-acetylglucosamine (UDPF) (1), SG8-UDPF (1:1) ionic complex (2), SG8-UDPF (2:1) ionic complex (3) and SG8-UDPF (4:1) ionic complex (4); and Fig. 1B shows morphological images of the cells.
Fig. 2A shows fluorescence images of cells treated with each of FITC-labeled vitagen (vitagen-FITC) (1), ARG6-vitagen-FITC ionic complex (2), ARG8-vitagen-FITC ionic complex (3), SG6-vitagen-FITC ionic complex (4) and SG8-vitagen-FITC ionic complex (5); Fig. 2B shows morphological images of the cells; and Fig. 2C shows merged images of A and B (Merge).
Fig. 3A shows fluorescence images of cells treated with each of FITC-labeled inositol phosphate (IPF) (1), ARG6-IPF ionic complex (2), ARG8-IPF ionic complex (3), SG6-IPF ionic complex (4) and SG8-IPF ionic complex (5); Fig. 3B shows morphological images of the cells; and Fig. 3C shows merged images of A and B.
Fig. 4A shows fluorescence images of cells treated with each of UDPF (1), ARG6-UDPF ionic complex (2), ARG8-UDPF ionic complex (3), SG6-UDPF ionic complex (4) and SG8-UDPF ionic complex (5); Fig. 4B shows morphological images of the cells; and Fig. 4C shows merged images of A and B.
Fig. 5A shows fluorescence images of cells treated with each of FITC-labeled 4-aminobenzoic acid (4-ABF) (1) and ARG8-4-ABF ionic complex (2); Fig. 5B shows morphological images of the cells; and Fig. 5C shows merged images of A and B.
Fig. 6 shows fluorescence images of cells at a depth of 45 µm under the skin, after being treated with each of vitagen-FITC (1), ARG8-vitagen-FITC ionic complex (2), SG8-vitagen-FITC ionic complex (3), 4-ABF (4), ARG8-4-ABF ionic complex (5) and SG8-4-ABF ionic complex (6) samples.
Fig. 7 shows fluorescence images of cells at a depth of 90 µm under the skin, after being treated with each of vitagen-FITC (1), ARG8-vitagen-FITC ionic complex (2), SG8-vitagen-FITC ionic complex (3), 4-ABF (4), ARG8-4-ABF ionic complex (5) and SG8-4-ABF ionic complex (6) samples.

### DETAILED DESCRIPTION OF THE INVENTION

In accordance with one aspect of the present invention, there is provided a composition for skin penetration, comprising an ionic complex in which a cationic compound of any one selected from the following compounds represented by formulae (1) to (4) and an anionic physiologically active material are combined through ionic bonding, the composition being used for delivering the physiologically active material into the skin: 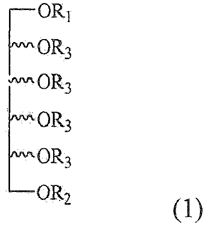 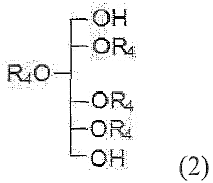 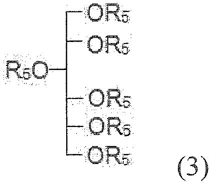 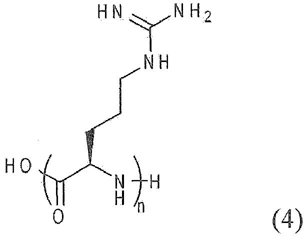 wherein
R₁ and R₂ are each independently H, C₁-C₆ alkyl, C₆-C₁₂ aryl C₁-C₆ alkyl, C₃-C₈ cycloalkyl, C₃-C₈ heteroalkyl, -(CH₂)ₘNHR', -(CH₂)₁CO₂R", -COR"', - SO₂R"", or a physiologically active material to be transported, where R', R", R"' and R"" are each independently H, C₁-C₆ alkyl, C₆-C₁₂ aryl C₁-C₆ alkyl, C₃-C₈ cycloalkyl, or C₃-C₈ heteroalkyl, m is an integer in the range of 2 to 5, and 1 is an integer in the range of 1 to 5;
R₃ is 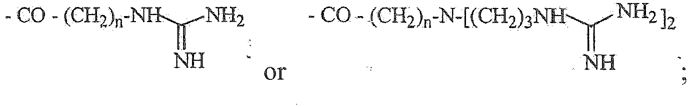
R₄ is 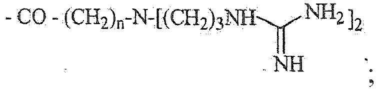
R₅ is 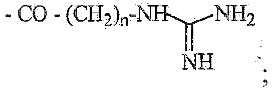 and
n is an integer in the range of 1 to 12.

Hereinafter, the present invention is described in more detail.

In an embodiment of the present invention, the ionic complex comprises a cationic compound.

The cationic compound may be any one of the compounds represented by the above formulae (1) to (4), and is used as a molecular transporter (*see* Korean Patent Nos. 10-0578732, 10-0699279, 10-0849033 and 10-1021078).

As shown in the above formulae (1) to (4), the cationic compound has a structure in which guanidine or arginine group having side chains of a variety of lengh is introduced into sugar or sugar-like backbone in linear or branch form, thereby exhibiting water solubility and excellent biomembrane permeability. Accordingly, the cationic compound can easily pass through the cell membrane and skin layer in the form of ionic complex with various anionic physiologically active materials such as diagnostic reagents, medicines, fluorescent materials, etc.

The compound of formula (1) has a structure in which 1 to 8 guanidine groups are introduced into the hydroxyl end of sugar alcohol derivatives, wherein desired functional groups can be introduced in high density into the backbone by using branches of dendrimer form. According to one embodiment of the present invention, n can be an integer in the range of 1 to 12 in the compound of formula (1). Preferably, the compound of formula (1) can be alditol derivatives or salts thereof having a steric conformation of sorbitol, mannitol or galactitol, for example.

In the compound of formula (2), n can be an integer in the range of 1 to 12. More preferably, the compound of formula (2) can be sorbitol derivatives or salts thereof having eight (8) guanidine groups introduced thereinto.

In the compound of formula (3), n can be an integer in the range of 1 to 12. More preferably, the compound of formula (3) can be sorbitol derivatives or salts thereof having six (6) guanidine groups introduced thereinto.

The compound of formula (4) can be arginine (in case n is 1) or an arginine oligomer (in case n is an integer in the range of 2 to 12). Preferably, the compound of formula (4) can be an arginine oligomer wherein n is an integer in the range of 6 to 8.

In an embodiment of the present invention, the ionic complex comprises an anionic physiologically active material.

The anionic physiologically active material may be a water-soluble molecule including a carboxylic acid group (-CO₂H), a phosphate group (-OP(O)(OH)₂) or a sulfonic acid group (-SO₃H). Preferably, the anionic physiologically active material can be selected from the group consisting of ascorbic acid phosphate, vitagen, inositol phosphate (IP), uridine diphosphate N-acetylglucosamine (UDP-GlcNAc), acetylsalicylic acid, 4-aminobenzoic acid (4-AB), hyaluronic acid, dextran sulfate and a combination thereof.

In addition, the anionic physiologically active material can be linked to a fluorescent material selected from the group consisting of fluorescein (FITC), dansyl (5-dimethylamino-1-naphthalene sulfonyl), rhodamine and a combination thereof.

According to one embodiment of the present invention, the anionic physiologically active material can be selected from the following materials:

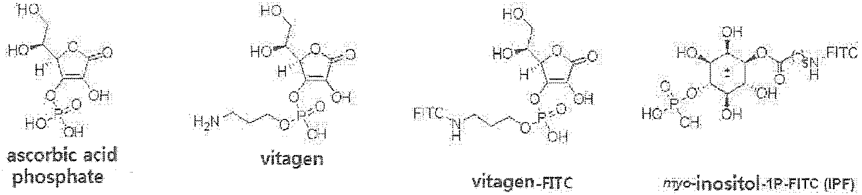

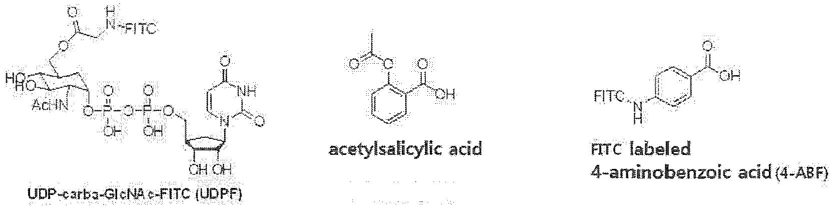

According to one embodiment of the present invention, the ionic complex is characterized in that the cationic compound and the anionic physiologically active material are combined through ionic bonding at a ratio of 1:1 to 4:1 on the basis of electric charges. The ratio of electric charges between the cationic molecular transporter compound and the anionic physiologically active material is critical to the manufacture of ionic complex for delivering molecules. When the ratio of cationic molecular transporter:anionic physiologically active material is 1:1 or higher, the formation of ionic complex with the substrate results in appropriate number of guanidine groups per molecule, which enhances cell permeability. When the ratio is 4:1 or lower, the competitive penetration through the cell membrane of the surplus molecular transporters in a free form, which are not involved in the formation of ionic complex with the substrate, can be prevented. As a result, the penetration rate of ionic complex may be increased and the efficiency of delivering a physiologically active material (substrate) may be improved.

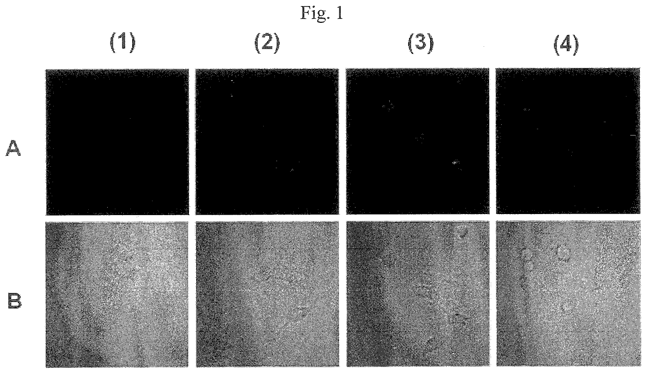

Therefore, the ionic complex of the present invention in which the cationic compound is combined with the anionic physiologically active material through ionic bonding at a ratio of 2:1 on the basis of electric charges is preferred (*see* Fig. 1).

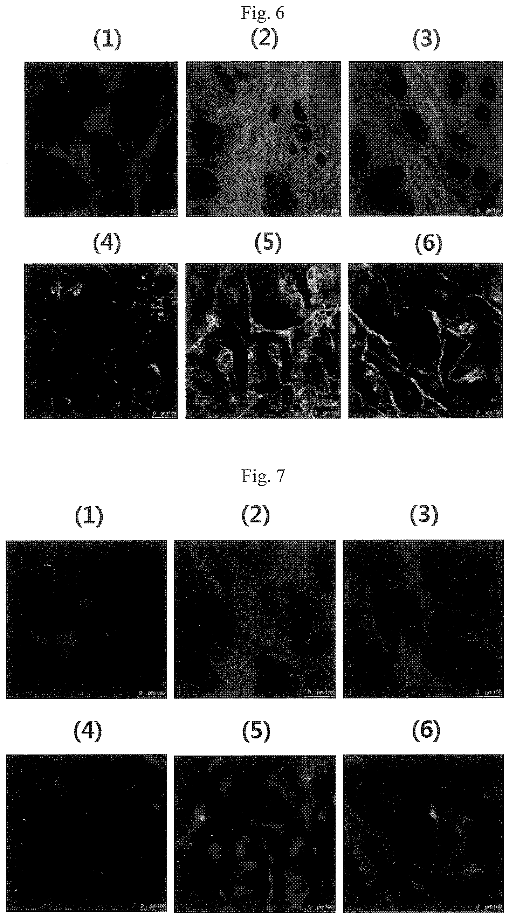

The composition for skin penetration according to the present invention is characterized by penetrating into the skin up to the depth of 125 to 200 µm, preferably, up to the depth of 150 to 200 µm. The depth corresponds to the stratum corneum, whole viable epidermis layer and the upper part of dermis layer of the skin. Therefore, the composition for skin penetration according to the present invention can penetrate into the cell or under the skin, e.g., between the epidermis layer and the dermis layer, to deliver physiologically active materials (*see* Test Example 2 and Figs. 6 and 7), and thus it can greatly improve the delivery of functional cosmetics and therapeutic agents for skin disorders. Furthermore, it can be appropriately used for transporting a variety of therapeutic or diagnostic agents as well as macromolecules, such as protein and gene, which requires subcutaneous administration.

The following Examples are intended to further illustrate the present invention without limiting its scope.

### Preparation Example: Preparation of fluorescence-labeled anionic substrate

### Preparation Example 1: Preparation of vitagen-FITC

Vitagen (99 mg, 0.316 mmol) was dissolved in a mixture of dimethylformamide and water (2.5:1 (v/v)) (2 mL), and then fluorescein-5-isocyanate (160 mg, 0.418 mmol) and triethylamine (300 µL, 2.158 mmol) were added thereto. The mixture was stirred at room temperature for 1 day. After completion of the reaction, the mixture was concentrated under a reduced pressure and then immediately purified by column chromatography (chloroform : ethanol : acetic acid = 16:3:1 to dichloromethane : methanol = 1:1) followed by ion exchange column chromatography using DOWEX 50WX8-400 ion exchange resin. The resultant was added with 1N NaOH to adjust the pH to 8-10, and then lyophilized to obtain the title compound (240 mg) as a yellow powder.

### Preparation Example 2: Preparation of 4-aminobenzoic acid-FITC (4-ABF)

4-Aminobenzoic acid (50 mg, 0.3308 mmol) was dissolved in a mixture of tetrahydrofuran and ethanol (3:2 (v/v)) (5 mL), and then fluorescein-5-isocyanate (154 mg, 0.397 mmol) and triethylamine (69 µL, 0.4962 mmol) were added thereto. The mixture was stirred at room temperature for 1 day. After completion of the reaction, the mixture was concentrated under a reduced pressure and then immediately purified by column chromatography (dichloromethane : methanol = 10:1) to obtain the title compound (145 mg) as a yellow powder.

### Example 1: Preparation of ionic complex comprising cationic compound having sorbitol backbone with eight (8) guanidine groups

### <1-1> Preparation of ionic complex comprising cationic compound having sorbitol backbone with eight (8) guanidine groups and vitagen-FITC

The cationic compound of formula (2) according to the present invention, having sorbitol backbone with eight (8) guanidine groups (+8; one positive charge per guanidine group) (hereinafter referred to as "sorbitol-based G8 molecular transporter" or "SG8"), was prepared by the process described in Example 1 of Korean Patent No. 10-0699279. Next, the prepared SG8 (52 mg, 30 µmol) was dissolved in 500 µL of triple distilled water to obtain cationic stock solution.

Vitagen-FITC (-1; one negative charge for one phosphate) (84 mg, 120 µmol) obtained in Preparation Example 1 was dissolved in 2 mL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "SG8-vitagen-FITC".

### <1-2> Preparation of ionic complex comprising SG8 and myo-inositol-1P-FITC

A cationic stock solution containing SG8 was prepared according to the process described in Example <1-1>.

*myo*-inositol-1P-FITC (hereinafter referred to as "IPF") (-1; one negative charge for one phosphate) (92 mg, 120 µmol) was dissolved in 2 mL of triple distilled water to obtain an anionic stock solution (K.C. Seo et al. Journal of Carbohydrate Chemistry, 2007, 26, 305-327).

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "SG8-IPF".

### <1-3> Preparation of ionic complex comprising SG8 and UDP-carba-GlcNAc-FITC (UDPF)

A cationic stock solution containing SG8 was prepared according to the process described in Example <1-1>.

UDP-carba-GlcNAc-FITC (hereinafter referred to as "UDPF") (-2; two negative charges for two phosphates) (66 mg, 60 µmol) was dissolved in 1 mL of triple distilled water to obtain an anionic stock solution (K.C. Seo et al. Chemical Communications, 2009, 1733-1735).

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "SG8-UDPF (2:1)".

### <1-4> Preparation of ionic complex comprising SG8 and UDPF

The procedure of Example <1-3> was repeated except that the cationic compound and the anionic physiologically active material were mixed at a ratio of 1:1 on the basis of electric charges to obtain an ionic complex comprising cationic compound : anionic physiologically active material at a ratio of 1:1 on the basis of electric charges.

The prepared ionic complex was called "SG8-UDPF (1:1)".

### <1-5> Preparation of ionic complex comprising SG8 and UDPF

The procedure of Example <1-3> was repeated except that the cationic compound and the anionic physiologically active material were mixed at a ratio of 4:1 on the basis of electric charges to obtain an ionic complex comprising cationic compound : anionic physiologically active material at a ratio of 4:1 on the basis of electric charges.

The prepared ionic complex was called "SG8-UDPF (4:1)".

### Example 2: Preparation of ionic complex comprising cationic compound having sorbitol backbone with six (6) guanidine groups

### <2-1> Preparation of ionic complex comprising cationic compound having sorbitol backbone with six (6) guanidine groups and vitagen-FITC

The cationic compound of formula (3) according to the present invention, having sorbitol backbone with six (6) guanidine groups (+6; one positive charge per guanidine group) (hereinafter referred to as "sorbitol-based G6 molecular transporter" or "SG6"), was prepared by the process described in Example 8 of Korean Patent No. 10-0699279. Next, the prepared SG6 (40 mg, 30 µmol) was dissolved in 500 µL of triple distilled water to obtain cationic stock solution.

Vitagen-FITC (63 mg, 90 µmol) obtained in Preparation Example 1 was dissolved in 1.5 mL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "SG6-vitagen-FITC".

### <2-2> Preparation of ionic complex comprising SG6 and IPF

A cationic stock solution containing SG6 was prepared according to the process described in Example <2-1>.

IPF (69 mg, 90 µmol) prepared by the process described in Example <1-2> was dissolved in 1.5 mL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "SG6-IPF".

### <2-3> Preparation of ionic complex comprising SG6 and UDPF

A cationic stock solution containing SG6 was prepared according to the process described in Example <2-1>.

UDPF (50 mg, 45 µmol) prepared by the process described in Example <1-3> was dissolved in 750 µL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "SG6-UDPF".

### Example 3: Preparation of ionic complex comprising arginine-octamer cationic compound

### <3-1> Preparation of ionic complex comprising arginine-octamer and vitagen-FITC

The cationic compound of formula (4) according to the present invention, arginine-octamer (hereinafter referred to as "ARG8") (n=8; +8), was obtained from Peptron Inc. Next, ARG8 (61 mg, 30 µmol) was dissolved in 500 µL of triple distilled water to obtain cationic stock solution.

Vitagen-FITC (84 mg, 120 µmol) obtained in Preparation Example 1 was dissolved in 2 mL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "ARG8-vitagen-FITC".

### <3-2> Preparation of ionic complex comprising ARG8 and IPF

A cationic stock solution containing ARG8 was prepared according to the process described in Example <3-1>.

IPF (92 mg, 120 µmol) prepared by the process described in Example <1-2> was dissolved in 2 mL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "ARG8-IPF".

### <3-3> Preparation of ionic complex comprising ARG8 and UDPF

A cationic stock solution containing ARG8 was prepared according to the process described in Example <3-1>.

UDPF (66 mg, 60 µmol) prepared by the process described in Example <1-3> was dissolved in 1 mL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "ARG8-UDPF".

### <3-4> Preparation of ionic complex comprising ARG8 and 4-ABF

A cationic stock solution containing ARG8 was prepared according to the process described in Example <3-1>.

4-ABF (-1; one negative charge for one carboxylate) (65 mg, 120 µmol) obtained in Preparation Example 2 was dissolved in 2 mL of triple distilled water containing 10% DMSO to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "ARG8-4-ABF".

### Example 4: Preparation of ionic complex comprising arginine-hexamer cationic compound

### <4-1> Preparation of ionic complex comprising arginine-hexamer and vitagen-FITC

The cationic compound of formula (4) according to the present invention, arginine-hexamer (hereinafter referred to as "ARG6") (n=6; +6), was obtained from Peptron Inc. Next, ARG6 (46 mg, 30 µmol) was dissolved in 500 µL of triple distilled water to obtain cationic stock solution.

Vitagen-FITC (63 mg, 90 µmol) obtained in Preparation Example 1 was dissolved in 1.5 mL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "ARG6-vitagen-FITC".

### <4-2> Preparation of ionic complex comprising ARG6 and IPF

A cationic stock solution containing ARG6 was prepared according to the process described in Example <4-1>.

IPF (69 mg, 90 µmol) prepared by the process described in Example <1-2> was dissolved in 1.5 mL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "ARG6-IPF".

### <4-3> Preparation of ionic complex comprising ARG6 and UDPF

A cationic stock solution containing ARG6 was prepared according to the process described in Example <4-1>.

UDPF (50 mg, 45 µmol) prepared by the process described in Example <1-3> was dissolved in 750 µL of triple distilled water to obtain an anionic stock solution.

The obtained anionic stock solution was slowly added to the cationic stock solution, and the mixture was stirred for 2-3 hours until the turbid mixture solution became clear. The mixture was lyophilized to obtain an ionic complex comprising cationic compound : anionic physiologically active material at a ratio of 2:1 on the basis of electric charges.

The prepared ionic complex was called "ARG6-UDPF".

### Test Example 1: Measurement of cell membrane permeability

In order to confirm the cell membrane permeability of the ionic complexes prepared in the above Examples, the fluorescence of FITC-labeled physiologically active material (substrate) was measured by Confocal Laser Scanning Microscope (Olympus FV1000).

First, HeLa cells (ATCC^{®} CCL-2™) were cultured in a dish plate. The cells were stabilized for 24 hours by using DMEM (Dulbecco's modified Eagle's medium) containing 10% fetal bovine serum before culturing on a serum-free medium for 24 hours to starve the cells. Thereafter, the cells were treated with the ionic complexes prepared in Examples 1-1 to 4-3 at a concentration of 48 µM (concentration of substrate) for 1 hour at 37°C. After the cells were washed with PBS (phosphate buffer solution) 5 times, the permeability through the cell membrane was immediately observed with a confocal microscope. In this case, each of vitagen-FITC, IPF, UDPF and 4-ABF (substrate) was used as a control.

An Ar laser (488 nm) was used for the excitation of fluorescence material and the cells were observed at 60X magnification. The results are shown in Figs. 1 to 5. In each of Figs. 1 to 5, column A shows fluorescence images of cells treated with samples, column B shows morphological images of the cells treated with samples, and column C shows merged images of the fluorescence images and the morphological images.

Fig. 1A shows fluorescence images of cells treated with each of UDPF (control) (1), SG8-UDPF (1:1) ionic complex of Example <1-4> (2), SG8-UDPF (2:1) ionic complex of Example <1-3> (3) and SG8-UDPF (4:1) ionic complex of Example <1-5> (4); and Fig. 1B shows morphological images of the cells.

As shown in Fig. 1, the cells treated with the ionic complexes of the present invention (Fig. 1A (2) to (4)) show much stronger fluorescence intensity than that treated with UDPF substrate only (Fig. 1A (1)). Especially, the cells treated with ionic complex comprising cationic compound : anionic physiologically active material at a ratio of 2:1 on the basis of electric charges (Fig. 1A (3)) shows the strongest fluorescence intensity. Accordingly, it is confirmed that the optimal ratio of electric charges for the ionic complex comprising cationic compound : anionic physiologically active material of the present invention is 4:1, preferably 2:1.

The above results indicate that when the ratio of molecular transporter (cation) is too low, the number of guanidine groups per molecule becomes relatively low due to the formation of ionic complex with substrate, which results in low cell penetration. Meanwhile, when the ratio of molecular transporter is too high, the surplus molecular transporters in a free form, which are not involved in the formation of ionic complex with substrate, penetrate the cell membrane competitively, which decreases the penetration rate of ionic complex.

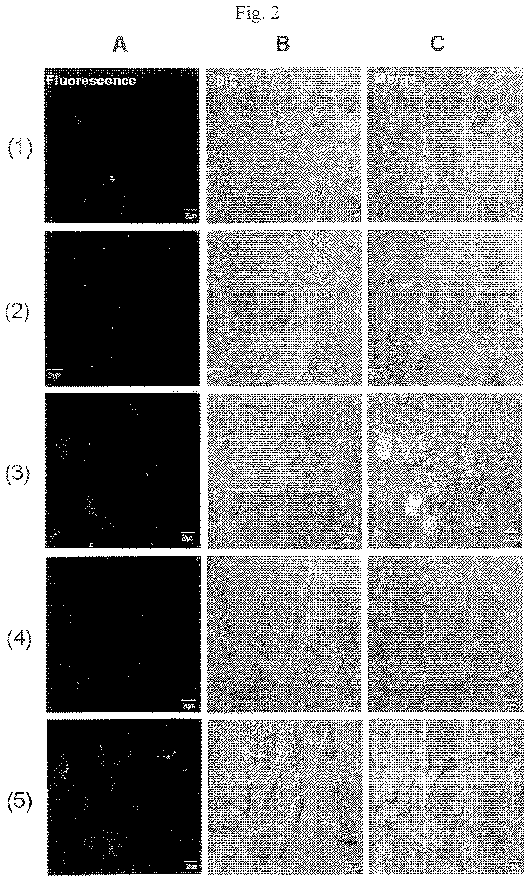

Fig. 2A shows fluorescence images of cells treated with each of vitagen-FITC (control) (1), ARG6-vitagen-FITC ionic complex of Example <4-1> (2), ARG8-vitagen-FITC ionic complex of Example <3-1> (3), SG6-vitagen-FITC ionic complex of Example <2-1> (4) and SG8-vitagen-FITC ionic complex of Example <1-1> (5); Fig. 2B shows morphological images of the cells; and Fig. 2C shows merged images of A and B.

As shown in Fig. 2, the cells treated with the ionic complexes of the present invention (Fig. 2A (2) to (5)) show much stronger fluorescence intensity than that treated with vitagen-FITC substrate only (Fig. 2A (1)). The results are caused by the enhanced cell membrane penetration due to the formation of ionic bonding between the anionic phosphate of vitagen substrate and a part of cationic guanidine groups of cationic compound (molecular transporter), and the residual guanidine groups of molecular transporter.

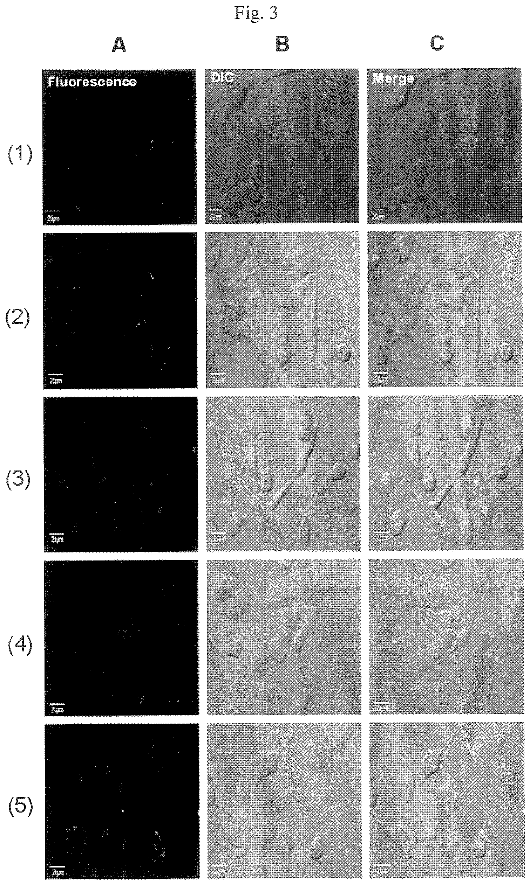

Fig. 3A shows fluorescence images of cells treated with each of IPF (control) (1), ARG6-IPF ionic complex of Example <4-2> (2), ARG8-IPF ionic complex of Example <3-2> (3), SG6-IPF ionic complex of Example <2-2> (4) and SG8-IPF ionic complex of Example <1-2> (5); Fig. 3B shows morphological images of the cells; and Fig. 3C shows merged images of A and B.

As shown in Fig. 3, the cells treated with the ionic complexes of the present invention (Fig. 3A (2) to (5)) show much stronger fluorescence intensity than that treated with IPF substrate only (Fig. 3A (1)). The results are caused by the enhanced cell membrane penetration due to the formation of ionic bonding between the anionic phosphate of IPF substrate and a part of cationic guanidine groups of cationic compound (molecular transporter), and the residual guanidine groups of molecular transporter.

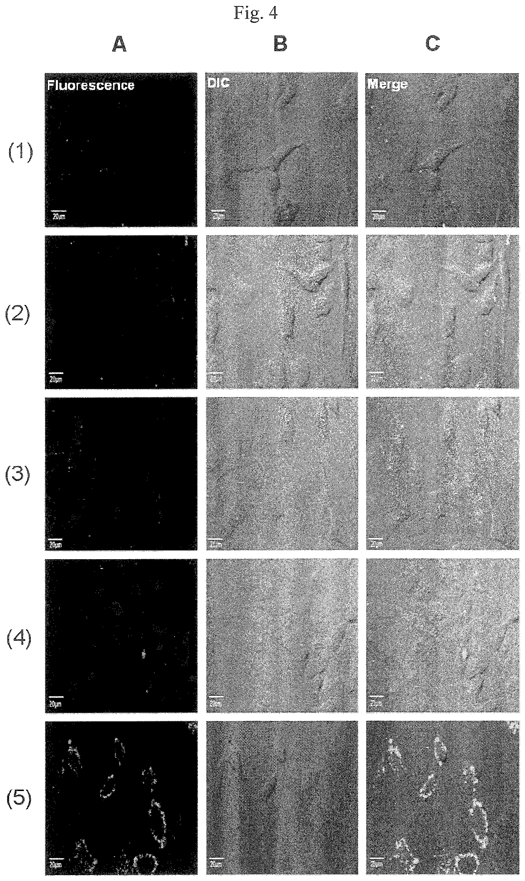

Fig. 4A shows fluorescence images of cells treated with each of UDPF (control) (1), ARG6-UDPF ionic complex of Example <4-3> (2), ARG8-UDPF ionic complex of Example <3-3> (3), SG6-UDPF ionic complex of Example <2-3> (4) and SG8-UDPF ionic complex of Example <1-3> (5); Fig. 4B shows morphological images of the cells; and Fig. 4C shows merged images of A and B.

As shown in Fig. 4, the cells treated with the ionic complexes of the present invention (Fig. 4A (2) to (5)) show much stronger fluorescence intensity than that treated with UDPF substrate only (Fig. 4A (1)). The results are caused by the enhanced cell membrane penetration due to the formation of ionic bonding between the anionic phosphate of UDPF substrate and a part of cationic guanidine groups of cationic compound (molecular transporter), and the residual guanidine groups of molecular transporter.

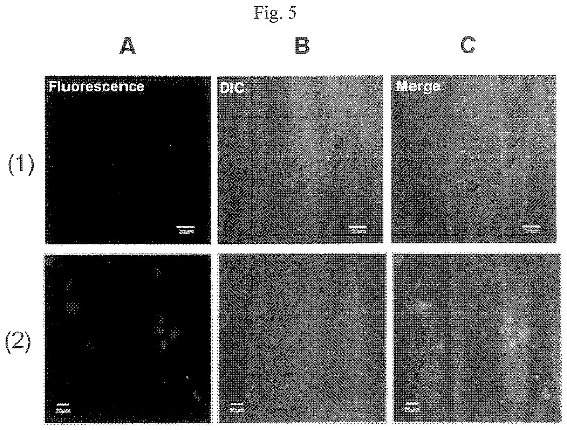

Fig. 5A shows fluorescence images of cells treated with each of 4-ABF (control) (1) and ARG8-4-ABF ionic complex of Example <3-4> (2); Fig. 5B shows morphological images of the cells; and Fig. 5C shows merged images of A and B.

As shown in Fig. 5, the cell treated with the ionic complex of the present invention (Fig. 5A (2)) shows much stronger fluorescence intensity than that treated with 4-ABF substrate only (Fig. 5A (1)). The results are caused by the enhanced cell membrane penetration due to the formation of ionic bonding between the anionic carboxylate of 4-ABF substrate and a part of cationic guanidine groups of cationic compound (molecular transporter), and the residual guanidine groups of molecular transporter.

### Test Example 2: Measurement of penetration into mouse skin and distribution therein

ARG8-vitagen-FITC ionic complex weighed in an amount of 16 mg of vitagen-FITC based on substrate, among 26 mg of ARG8-vitagen-FITC ionic complex prepared in Example <3-1>, was dissolved in 84 µL of water, and mixed with 300 mg of polyethylene glycol (PEG) 400 to prepare 4% sample solution.

Each of SG8-vitagen-FITC ionic complex prepared in Example <1-1> and ARG8-4-ABF ionic complex prepared in Example <3-4> was used to prepare each of 4% sample solution, according to the process described above.

Four-week old BALB/c nude mice were anesthetized with gas, and 50 µL of each sample solution as prepared above was applied to the skin of the femoral region of the hind leg in an area of 1cm×1cm. The mice were placed in a dark room under anesthetic condition for 3 hours. After washing the sample applied to the leg with distilled water and 70% ethanol, and the mice were euthanized with carbon dioxide. The dermal tissue of the femoral region was exfoliated, placed on a slide glass, and fixed with cover slip. Transdermal permeability of each sample on the slide glass was observed with Two-photon Laser Scanning Microscope (Leica), and femto-second laser (960 nm) was used for the excitation of fluorescence material. The subcutaneous tissue was continuously photographed at an interval of 2 µm depth, and the images of representative depth (45 µm and 90µm) were observed.

Fig. 6 shows fluorescence images of cells at a depth of 45 µm under the skin, after being treated with each of 4% vitagen-FITC (control) (1), 4% ARG8-vitagen-FITC ionic complex (2) and 4% SG8-vitagen-FITC ionic complex (3); and 4% 4-ABF (control) (4), 4% ARG8-4-ABF ionic complex (5) and 4% SG8-4-ABF ionic complex (6) samples.

Furthermore, Fig. 7 shows fluorescence images of cells at a depth of 90 µm under the skin, after being treated with each of 4% vitagen-FITC (control) (1), 4% ARG8-vitagen-FITC ionic complex (2) and 4% SG8-vitagen-FITC ionic complex (3); and 4% 4-ABF (control) (4), 4% ARG8-4-ABF ionic complex (5) and 4% SG8-4-ABF ionic complex (6) samples.

As shown similarly in Figs. 6 and 7, the ionic complex samples of the present invention in which each substrate and molecular transporter compound are combined through ionic bonding can effectively penetrate into the skin of mice, compared with the control group of vitagen-FITC or 4-ABF. Furthermore, it is observed that the amount of physiologically active materials reduces with the depth of the skin, which results in weakened fluorescence intensity.

Furthermore, the results of transdermal penetration measured by Two-photon Laser Scanning Microscope (Leica) show that the fluorescence-labeled physiologically active material penetrated into the depth of at least 125 to 200 µm, which corresponds to the stratum corneum, whole viable epidermis layer and the upper part of dermis layer of the skin.

As a result, it is confirmed that the ionic complex prepared according to the present invention exhibits an excellent permeability to the cell membrane and a superior transdermal penetration. Accordingly, the ionic complex of the present invention can be effectively used for delivering water-soluble anionic physiologically active materials into the cell or under the skin (viable epidermis layer and dermis layer).

While the invention has been described with respect to the above specific embodiments, it should be recognized that various modifications and changes may be made to the invention by those skilled in the art which also fall within the scope of the invention as defined by the appended claims.

## Claims

1. A composition for skin penetration, comprising an ionic complex in which a cationic compound of any one selected from the following compounds represented by formulae (1) to (4) and an anionic physiologically active material are combined through ionic bonding, the composition being used for delivering the physiologically active material into the skin: 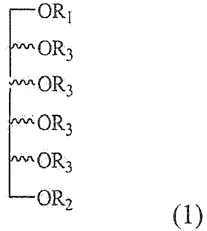 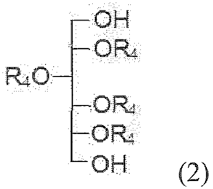 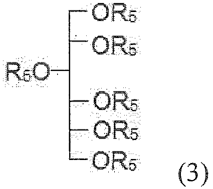 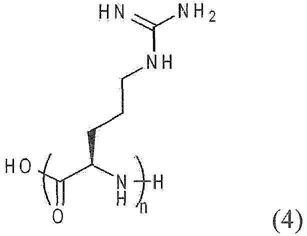 wherein
R₁ and R₂ are each independently H, C₁-C₆ alkyl, C₆-C₁₂ aryl C₁-C₆ alkyl, C₃-C₈ cycloalkyl, C₃-C₈ heteroalkyl, -(CH₂)ₘNHR', -(CH₂)₁CO₂R", -COR"', - SO₂R"", or a physiologically active material to be transported, where R', R", R"' and R"" are each independently H, C₁-C₆ alkyl, C₆-C₁₂ aryl C₁-C₆ alkyl, C₃-C₈ cycloalkyl, or C₃-C₈ heteroalkyl, m is an integer in the range of 2 to 5, and 1 is an integer in the range of 1 to 5;
R₃ is 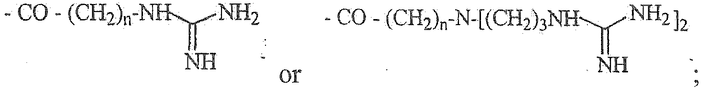 R₄ is 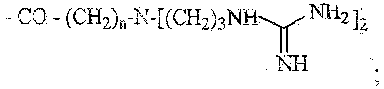
R₅ is 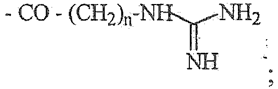 and
n is an integer in the range of 1 to 12.

2. The composition for skin penetration of claim 1, wherein, in the ionic complex, the cationic compound is combined with the anionic physiologically active material at a ratio of 1:1 to 4:1 on the basis of electric charges.

3. The composition for skin penetration of claim 1, wherein the anionic physiologically active material is a water-soluble molecule including a carboxylic acid group (-CO₂H), a phosphate group (-OP(O)(OH)₂) or a sulfonic acid group (-SO₃H).

4. The composition for skin penetration of claim 3, wherein the anionic physiologically active material is selected from the group consisting of ascorbic acid phosphate, vitagen, inositol phosphate (IP), uridine diphosphate N-acetylglucosamine (UDP-GlcNAc), acetylsalicylic acid, 4-aminobenzoic acid (4-AB), hyaluronic acid, dextran sulfate and combinations thereof.

5. The composition for skin penetration of claim 3, wherein the anionic physiologically active material is linked to a fluorescent material selected from the group consisting of fluorescein (FITC), dansyl (5-dimethylamino-1-naphthalene sulfonyl), rhodamine and combinations thereof.

6. The composition for skin penetration of claim 1, wherein the composition penetrates between the epidermis layer and the dermis layer of the skin.

7. The composition for skin penetration of claim 6, wherein the composition penetrates into the skin up to the depth of 125 to 200 µm.
